# EUROPÄISCHE PATENTSCHRIFT

(11) **EP 1 965 758 B1**
(45) Veröffentlichungstag und Bekanntmachung des Hinweises auf die Patenterteilung: **07.03.2012**
(21) Anmeldenummer: 06828856.2
(22) Anmeldetag: 24.10.2006
(51) Int. Cl.: A61K 8/64, A61K 8/68, A61P 17/00, A61K 9/107

(54) **ZUSAMMENSETZUNGEN, ENTHALTEND PROTEINE ZUM TRANSFER / RECYCLING STRUKTURELL VERÄNDERTER LIPIDE SOWIE DEREN ANWENDUNGEN**
COMPOSITIONS CONTAINING PROTEINS FOR THE TRANSFER/RECYCLING OF STRUCTURALLY MODIFIED LIPIDS, AND THE APPLICATIONS THEREOF
COMPOSITIONS CONTENANT DES PROTEINES POUR LE TRANSFERT/RECYCLAGE DE LIPIDES STRUCTUELLEMENT MODIFIES ET LEURS APPLICATIONS

(30) Priorität: 28.11.2005 DE 102005056538
(43) Veröffentlichungstag der Anmeldung: 10.09.2008
(73) Patentinhaber: Merz Pharma GmbH & Co. KGaA, 60318 Frankfurt Main (DE)
(72) Erfinder: BEUTLER, Rolf, D., 64739 Höchst/Hummetroth (DE); SCHMIDT,Karlheinz, 72810 Gomaringen (DE)
(74) Vertreter: Müller, Claudia
(86) Internationale Anmeldenummer: PCT/EP2006/010223
(87) Internationale Veröffentlichungsnummer: WO 2007/059836

(56) Entgegenhaltungen:
- WO-A-01/40370
- WO-A-02/15866
- WO-A-02/055048
- WO-A-2004/105716
- DE-A1- 3 815 473
- ALTAMURA N ET AL: "Effect of N-ethylmaleimide on rat liver phosphatidylcholine-specific and non-specific transfer protein activities: its dependence on donor liposome composition." THE INTERNATIONAL JOURNAL OF BIOCHEMISTRY 1986, Bd. 18, Nr. 6, 1986, Seiten 513-517, XP009087562 ISSN: 0020-711X

## Beschreibung

### Gegenstand der Erfindung

Die vorliegende Erfindung betrifft Zusammensetzungen, enthaltend Recycling-Transferproteine für strukturell / funktionell veränderte Lipide, ein Lipiddepot aus strukturell nicht veränderten Lipiden und nicht transferierbare Wirkstoffe. Derartige Zusammensetzungen können zum Engineering von Biomembranen, z. B. durch äußere topische Anwendung, insbesondere auf Haut / Schleimhaut, eingesetzt werden. Dabei werden strukturell bzw. funktionell, vor allem oxidativ veränderte Lipide aus den diese enthaltenden Strukturen wie z. B. Lipidaggregaten der Haut, entfernt, extern in einem Depot regeneriert und vorzugsweise wieder in die Strukturen zurückgeführt. Es handelt sich also um ein Recycling veränderter Lipide aus Biomembranen. Die Zusammensetzungen sind insofern autoregulativ und können vor allem kosmetisch, kosmetisch- balneologisch oder auch dermatologisch/ pharmazeutisch angewendet werden.

### Hintergrund der Erfindung

Lipide haben eine große strukturelle und funktionelle Bedeutung als Bestandteile von technischen Produkten, Bedarfsgegenständen, Nahrungs- und Genußmitteln, Arzneimitteln, Diagnostika, Kosmetika, Hygiene- und Medizinprodukten. Eine besondere Rolle kommt Lipiden auch in biologischen Materialien zu, wo sie unter anderem als Energieträger, als Wärme- und Elektro-Isolatoren, Diffusions-Evaporationsbarrieren, Signal-, Farb- Schutzstoffe, Kofaktoren von Enzymen oder auch als strukturgebende Elemente von Organellen, Zellen, Organen und Organismen Verwendung finden.

Dem Fachmann sind zahlreiche Lipidklassen bekannt wie zum Beispiel Fettsäuren, Fettalkohole, Ester- und Ether Lipide, Steroide. Diese strukturell verschiedenen Lipidklassen können unterschiedliche physikalische und chemische Eigenschaften aufweisen. So besitzen amphiphile Lipide wie die in biologischen Materialien weit verbreiteten Phospholipide die Eigenschaft, in Gegenwart von Wasser leicht Aggregate, Cluster oder Phasen (Mizellen, Mono-Bi- Multilayer, Liposomen, Röhren) mit ganz spezifischen strukturellen und funktionellen Eigenschaften zu bilden. Bei der Bildung höherer Aggregate aus amphiphilen Lipiden können auch weitere Substanzen wie Proteine, Kohlenhydrate, andere Biopolymere oder technische Polymere mitwirken und die Eigenschaften zusätzlich modifizieren. Die in biologischen Materialien, aber auch in Lebensmitteln, Arzneimitteln oder technischen Produkten enthaltenen Lipidaggregate weisen ungesättigte Lipide auf, die für die Funktion der Aggregate wichtig sind. Diese können jedoch leicht durch Luftsauerstoff, UV Licht, chemische Oxidantien, freie Radikale oxidiert werden. Ein Beispiel ist das "Ranzigwerden" von ungesättigten Speisefetten. Die Oxidation verläuft dabei häufig in Radikalkettenreaktionen, deren Stillstand meist erst mit dem Verbrauch der Substrate erreicht ist. Auch andere strukturelle und damit funktionelle Änderungen bzw. Beeinträchtigungen an Lipiden sind möglich wie z. B. Isomerisierung, Bildung höherer Aggregate, Oligo- Polymerisierung. Die bei der Veränderung von Lipiden, insbesondere bei der oxidativen Veränderung entstehenden Reaktions- oder Oxidationsprodukte sind nicht selten toxisch, vor allem im Organismus. Die Oxidation von Lipid- Aggregaten biologischer Membranen wird daher auch im Zusammenhang mit dem Prozess der biologischen Alterung in Verbindung gebracht, wobei einige Endprodukte der Oxidation wie Ceroide oder Lipofuscine im Gewebe eingelagert und als Alterspigmente bezeichnet werden.

Bisher wurde zur Verhinderung insbesondere oxidativer Lipidveränderungen versucht, den die Lipide enthaltenden Strukturen anti- oxidativ wirkende Substanzen zuzuführen. Hierzu gehören beispielsweise phenolische Substanzen, die mittels lipophiler Isoprenreste in den Lipidaggregaten ankern und bei oxidativem Angriff selbst in die chinoide Form übergehen, vgl. auch DE 199 62 369 A1. Je höher die Konzentration an anti- oxidativen Substanzen, umso wirksamer der Schutz vor Lipidveränderung. Das Problem der wirksamen Inkorporierung lipophiler Stoffe in Zellaggregate kann dabei gemäß der DE 38 15 473 C1 mittels sogenannter Transferproteine gelöst werden. Dieses System wird auch gemäß der DE 103 24 256 A1 eingesetzt, um durch eine topische Anwendung auf der Haut unter Verzicht auf unerwünschte Emulgatorkomponenten die Inkorporierung oder den Austausch von Lipiden in den diese enthaltenden Strukturen zu bewirken. Dabei werden die vorhandenen Lipide selbst nicht in ihrer Funktion geändert, sondern durch andere Lipde ergänzt oder ersetzt. Es handelt sich hier also um den Transfer strukturell / funktionell bzw. oxidativ nicht veränderter Lipide. So kann zum Beispiel Vitamin E als lipophile anti- oxidative Substanz in Lipidaggregate inkorporiert werden. Bei mäßigen oxidativen Belastungen können moderate Konzentrationen an derartigen Antioxidativa einen gewissen Schutz gegen die oxidative Alterung aufbauen. Bei stärker oxidativen Belastungen und höheren Konzentrationen an Antioxidantien reichern sich jedoch die oxidierten Folgeprodukte in den Lipidaggregaten an und können dort selbst zu Funktionsstörungen führen. Die oben erwähnten Transferproteine für nicht veränderte Lipide können analog auch gemäß der DE 10 2004 057 150 A1 zusammen einem Kapillar- Wirksystem eingesetzt werden. Gemäß der DE 693 00 514 T2 werden bestimmte Cholesterin- reduzierte und damit transparente Vesikeldispersionen zum Wirkstofftransport vorgeschlagen. In der DE 694 00 746 T2 werden Kombinationen aus 2 Lipiddispersionen als Wirkstoffträger für die Behandlung sowohl von äußerer als auch innerer Hautschicht vorgeschlagen. Gemäß der DE 697 30 604 T2 werden Oxadisäuren eingesetzt, um Störungen der Haut, wie z.. B. Altershaut zu behandeln. Pigmentierungsstörungen z. B. bei Altershaut sollen nach der DE 698 13 935 T2 mittels Phenolgruppen haltigen Retinoiden behandelt werden.

### Aufgabe vorliegender Erfindung

Der vorliegenden Erfindung liegt daher die Aufgabe zugrunde, den oben beschriebenen Mängeln abzuhelfen und ein System bereitzustellen, mit welchem der strukturellen und funktionellen Beeinträchtigung von Lipidaggregaten wirksam begegnet werden kann, ohne dass es zur Anlagerung unerwünschter schädlicher Folge- oder Reaktionsprodukte kommt.

### Lösung der Aufgabe

Diese Aufgabe wird erfindungsgemäß gelöst durch Zusammensetzungen, enthaltend
a) ein Depot aus einem oder mehreren strukturell/ funktionell nicht veränderten Lipiden;
b) ein oder mehrere Recycling-Transferproteine mit Transferraten für strukturell / funktionell veränderte Lipide,
c) einen oder mehrere nicht transferierbare Wirkstoffe zur Regenerierung strukturell/ funktionell veränderter Lipide;
d) ggf. einen oder mehrere anwendungsbezogene Zusatzstoffe.

Insbesondere handelt es sich bei den Recycling-Transferproteinen um solche mit Transferraten für oxidativ veränderte Lipide und bei den Wirkstoffen um solche zur Regenerierung oxidativ veränderter Lipide, wobei diese Lipide vor allem biologischen Ursprungs sind. Die Wirkstoffe zur Regenerierung, vor allem oxidativ veränderter Lipide, sind nicht transferierbar, so dass eine Anreicherung von dadurch entstandenen Folgeprodukten in den Lipidstrukturen oder Aggregaten vermieden werden kann. Somit kann das strukturell und vor allem oxidativ veränderte Lipid, insbesondere biologischen Ursprungs, mittels des dafür vorgesehenen Transferproteins aus den diesen enthaltenden Aggregaten herausgelöst, in das Depot mit nicht veränderten, vor allem oxidativ nicht veränderten Lipiden transferiert, und durch die hier vorhandenen Wirkstoffe regeneriert sowie anschließend rücktransferiert werden. Überraschenderweise erfolgt insofern durch die externe Regenerierung eine unerwartete Steigerung der Effizienz von gegen die strukturelle Veränderung, vor allem Oxidation von Lipiden gerichteten Maßnahmen. Die Energiebarriere, welche zwischen den beiden Kompartimenten, das heißt dem Lipidaggregat mit strukturell veränderten Lipiden einerseits und dem Depot andererseits, besteht und den Austausch über die durch die thermodynamische Triebkraft bewirkte Gleichverteilung der strukturell veränderten Lipide hinaus behindert, kann mit Hilfe der Transferproteine überwunden werden.

Besonders geeignete Zusammensetzungen enthalten Transferproteine bzw. - proteinhydrolysate der beschriebenen Art aus tierischen, pflanzlichen oder marinen Quellen, ggf. auch aus mikrobiologischen Quellen, wie Getreide, Knollenfrüchten, Milch, Seide, mit den Merkmalen wie nachfolgend erläutert.

In einer weiteren Ausführungsform werden zusätzlich Transferproteine mit Transferaktivität für nicht strukturell funktionell veränderte Lipide gemäß Stand der Technik (DE 38 15 473 C1 oder DE 103 24 256 A1) eingesetzt, was zu einem erhöhten katalytischen Transfer nicht veränderter Lipide führt.

Die Lipide sind vor allem ausgewählt aus natürlichen oder synthetischen Fetten Ölen, Wachsen, Fettalkoholen, Fettsäureestern, Fettsäurepartialestern, Glyceriden, Silikonölen, Silikonwachsen, Kohlenwasserstoffen. Lecithinen, Sphingolipiden, Cholesterinen, Phospholipiden, Gangliosiden, Cerebrosiden, Ceramiden oder Mischungen hiervon. Ganz besonders geeignet als Lipiddepot ist eine Fettphase in Form uni - oder multilamellarer Liposomenen, welche sich aus den genannten Substanzen ergeben, nämlich Lecithinen, Sphingolipiden, Phospholipiden, Ceramiden oder Mischungen hiervon.

Besonders zum Recycling geeignete Wirkstoffe sind vor allem nicht transferierbare, insbesondere wasserlösliche oder wasserdispergierbare Wirkstoffe wie höhermolekulare Enzymproteine (z. B. Peroxidasen), Kofaktoren wie Gluthathion, alpha-Liponsäure, wasserlösliche Antioxidantien wie Ubichinone, Redoxsysteme wie Erythorbinsäure.

Darüber hinaus können auch pflanzliche Phenole wie z. B. Quercetin, Apfelquercetin, Cystus- Tee-Extrakt (aus Cystus incanus ssp. Tauricus) als nicht transferierbare Wirkstoffe eingesetzt werden. Insbesondere bevorzugt als nicht transferierbare Wirkstoffe sind Peroxidasen, Erythorbinsäure, Gluthation, auch Cystus-Tee- Extrakt, Apfelquercetin oder Mischungen hiervon.

Bevorzugt weisen die Zusammensetzungen für die Anwendungsform geeignete Zusatzstoffe, bevorzugt für die topische Anwendung, ausgewählt aus Wasser und Additiven, hierunter insbesondere, grenzflächenaktive Stoffe (wie O/W- W/O-Co-Emulgatoren, Co-Tenside oder Mischungen), Kolloide natürlicher oder synthetischer Herkunft, Konsistenzregler, Farb-Parfüm- Konservierungsstoffe, Pflegestoffe sowie Zusatzwirkstoffe oder Mischungen hiervon auf.

### Nähere Beschreibung der Zusammensetzungen

Die erfindungsgemäßen Zusammensetzungen sind für die topische Applikation bzw. Anwendung auf Schleimhaut konzipiert und umfassen eine Fett- bzw. Ölphase mit geeigneten Lipiden für das Lipiddepot, ein oder mehrere zum Transfer veränderter Lipide geeignete Recycling -Proteine, ein oder mehrere zum Recycling geeignete, nicht transferierbare Wirkstoffe, sowie für die gewünschte Anwendung geeignete Zusatzstoffe. Diese sind vorzugsweise ausgewählt aus galenisch und / oder pharmazeutisch akzeptablen Additiven und Wasser. Es kann insofern ein gezieltes Engineering von Biomembranen in vivo, ex vivo, in vitro oder von künstlich hergestellten Lipid- Membranen erfolgen. Die Anwendung kann insbesondere kosmetisch, dermatologisch, pharmazeutisch, kosmetischbalneologisch unter Einsatz einer jeweils geeigneten Applikationsform sein. Die erfindungsgemäßen Zusammensetzungen können vorzugsweise in Form einer Creme (z. B. O/W, W/O, Gelcreme), Lotion, in Form eines Gels, Balsams, Sprays, einer Maske oder Schaumes vorliegen. So können vor allem Mittel im Sinne einer Anti- Age Kosmetik, z. B. durch Auftragen auf die Haut, oder Einwirken auf die Haut bei Bade- oder Duschanwendungen bereitgestellt werden. Darüber hinaus können die Mittel auf Schleimhäute z. B. zur Anwendung bei Plaques und Läsionen eingesetzt werden.

Nachfolgend werden die einzelnen Komponenten der Zusammensetzungen erläutert.

### 1) Proteine für den Lipid-Transfer

Wegen der geringen Wasserlöslichkeit von Lipiden erfolgt der Austausch einzelner Lipidmoleküle zwischen Lipidaggregaten in einem wässrigen Medium langsam, d. h. wenig effizient und im wesentlichen aufgrund der thermischen Bewegung der Moleküle. Mittels Lipidtransferproteinen kann die Aktivierungsenergie zwischen den Lipid- Aggregaten katalytisch abgesenkt werden (vgl. auch DE C1 38 15 473).

Erfindungsgemäß einzusetzende Recycling - Proteine werden im allgemeinen aus pflanzlichen, tierischen, gentechnischen oder ggf. mikrobiellen Quellen erhalten und weisen insgesamt, je nach Herkunft, eine mittlere Molekülmasse von etwa 2500 bis 40 000 D auf. Dabei weisen pflanzliche Proteine aus Getreide wie Hafer, Hirse, Weizen, Mais, Gerste, Dinkel eine mittlere Molekülmasse von 4000 bis 8000 D, Proteine aus Knollenfrüchten oder Früchten eine mittlere Molekülmasse von 6000 bis 20 000 D, Proteine aus Milch eine mittlere Molekülmasse von 2500 bis 8000 D, Proteine aus Seide, Mikroorganismen, marinen Quellen eine mittlere Molekülmasse von 10 000 D bis 40000 D, vor allem 15000 D bis 40 000 D auf. Es können auch Mischungen eingesetzt werden. Die genannten Proteine können auch gentechnisch hergestellt werden.

Als Knollenfrüchte eignen sich Kartoffeln, Ylang Ylang, Topinambur oder ähnliche. Marine Quellen sind beispielsweise Algen, Muscheln, Meeresfrüchte. Geeignete Mirkoorganismen sind z. B. Bakterien. Bevorzugt sind Proteine pflanzlicher, tierischer Herkunft und aus marinen Quellen, wobei erstere besonders bevorzugt sind. Bei marinen Produkten sind insbesondere auch Algenproteine bevorzugt, vor allem solche mit einem mittleren MW von 12000 bis 25000 D.

Pflanzliche Proteine sind insbesondere solche aus Getreide, insbesondere Dinkel, Hirse, Hafer, Mais, Soja, tierische vor allem solche aus Milch, Seide.

Besonders bevorzugte Proteine werden ausgewählt aus pflanzlichen Proteinen, insbesondere Hafer, Weizen, Mais, Gerste, Soja, Bohnen, Hirse, Dinkel, Buchweizen, Tapioka, Topinambur, Kartoffeln, oder Mischungen hiervon. Insbesondere bevorzugt sind Hirse, Dinkel, Buchweizen, Tapioka; oder auch Hafer, Mais, Gerste, Weizen; oder Mischungen hiervon.

Die zum Transfer geeigneten Proteine bzw. Hydrolysate können durch Aufschlämmung in Wasser, ggf. Umsetzen mit Säuren oder Basen, weitere Aufarbeitung wie Zentrifugation, ggf. Abtrennung von Begleitstoffen durch Fällung z. B. mit Säure, Salzen (wie Ammoniumsulfat), Entfettung mit organischen Lösungsmitteln, Reinigung durch Dialyse, Gelfiltration, Chromatographie etc. gewonnen werden. Verfahren dazu sind im Stand der Technik bekannt und beispielsweise in der DE 38 15 473 C1 beschrieben, siehe auch US A 5 776 470, US A 6 077 529.

Die erfindungsgemäß eingesetzten Proteine verfügen über eine Transferrate für strukturell veränderte, insbesondere oxidativ veränderte Lipide. Diese Transferrate lässt sich im Resonanz Transfer- Energie Test (RET- Test) bestimmen, vgl. Nichols, J.W., Pagano R.E., J. Biol. Chem. 258 (1983), 5368-5371. Dazu können als Lipidaggregat auf herkömmliche Weise (z. B. durch Detergenzdialyse und Größenbestimmung durch Streulichtmessung) gewonnene Liposomen mit ungesättigten Lipiden wie z. B. 10% Dioleyl-phophatidylcholin, die zwei verschiedene Fluoreszenzmarker tragen, wie z. B. N-NBD (N-[7-Nitro-2,1,3-benzoxadiazol-4-yl] und N-Rh(N-[lissamin Rhodamin B]) verwendet werden. Die Lipide werden an den Doppelbindungen nach bekannten Verfahren oxidiert, z. B. mit Luftsauerstoff und einem Radikalkettenstarter wie 1,1'-Azo-bis-(cyclohexan-carbon-säurenitril). Der Grad der Oxidation kann auf bekannte Weise wie Dünnschichtchromatographie der Phospholipide oder nach Hydrolyse durch Gaschromatographie der Fettsäuremethylester bestimmt werden. Anhand des durch die eingesetzten Proteine bewirkten Transfers der fluoreszenzmarkierten Lipide aus den Donorliposomen in die Akzeptorliposomen erfolgt die Bestimmung der katalytischen Aktivität der Proteine.

Überraschenderweise zeigte sich, dass die erfindungsgemäßen Systeme dann wirksam sind, wenn die spezifische Aktivität der Recycling-Transferproteine für die genannten, insbesondere oxidierten Lipide zwischen 10 und 10000 ng/min/mg Protein, insbesondere 150 bis 10000, und bevorzugt 500 bis 9000 und insbesondere 800 bis 8000ng/min/mg Protein beträgt.

Besonders bevorzugt sind erfindungsgemäße Recycling- Transferproteine für veränderte Lipide, welche eine wie oben angegebene Transferrate, bevorzugt 600 bis 8000ng/min/mg Protein, eine mittlere Molekülmasse von 3500 bis 8000 D aufweisen und aus pflanzlichen Proteinen, insbesondere Getreide und hierunter vor allem Hafer, Weizen, Mais, Gerste, Hirse, Dinkel; oder aus Soja, Kartoffeln, Topinambur mit einer mittleren Molekülmasse von 6000 D bis 15000 D stammen. Eine weitere bevorzugte Gruppe erfindungsgemäß geeigneter Transferproteine sind solche aus Milch mit einer mittleren Molekülmasse von 2500 bis 8000 D, oder aus Seide mit einer mittleren Molekülmasse von 15000 bis 20000, mit jeweils einer Transferrate wie angegeben, bevorzugt von 500 bis 7000 ng/min/mg Protein.

Auch Mischungen der vorgenannten bevorzugten pflanzlichen und tierischen Proteine sind besonders geeignet.

Auch Proteine mit den genannten geeigneten Transferraten aus marinen Quellen können gewählt werden, vor allem aus Algen , Muscheln, Korallen. Deren mittlere Molekülmasse beträgt insbesondere 7500 D, die Transferrate ist bevorzugt hier 300 bis 8500 ng/min/mg Protein.

Auf mikrobiologischem Weg gewonnene Proteine stammen beispielsweise aus Pilzkulturen, und weisen die genannte Transferrate und eine mittlere Molekülmasse von 15000 D bis 25000 D auf.

Ganz besonders bevorzugt sind pflanzliche Proteine wie beschrieben, tierische Proteine aus Seide oder vor allem aus Milch wie oben beschrieben. Auch Mischungen von Proteinen aus diesen beiden Gruppen sind besonders geeignet. Insbesondere sind die erfindungsgemäß eingesetzten Proteine zumindest wasserdispergierbar, vor allem aber wasserlöslich. Sie wirken vor allem als Transfervehikel. Sie können in Mengen von 0,01 bis 10 Gew.%, vor allem 0,01 bis 8 Gew. %, eingesetzt werden.

Bevorzugt werden Mengen von 0,01 bis 5 Gew. %, insbesondere 0,01 bis 3 Gew. % , vor allem 0,01 bis 0,95 Gew.%, und ganz besonders 0,01 bis 0,85 oder auch 0,1 bis 0,85, insbesondere 0,1 bis 0,5 Gew.% gewählt.

Es können auch Kombinationen von Vertretern aus den verschiedenen Gruppen eingesetzt werden, insbesondere aus tierischen Proteinen und pflanzlichen Proteinen der genannten Art. Dabei kann das Verhältnis der Substanzen aus den unterschiedlichen Gruppen variiert werden z. B. von 1:4 bis 1:10, insbesondere 1:1 bis 1:6 erfolgen. Vor allem geeignet ist Milchprotein, insbesondere hydrolysiertes Milchprotein in Kombination mit einem Pflanzenprotein, ausgewählt aus Haferprotein, Gersteprotein, Hirseprotein, Dinkelprotein wie beschrieben, vor allem mit bevorzugten Molekülmassen, oder marinen Proteinen im jeweils angegebenen insbesondere bevorzugten Transferraten- und Molekulargewichtsbereich.

Gegebenenfalls katalysieren bestimmte Recycling - Proteine der geschilderten Art, welche die angegebenen Molekülmassen und Transferraten aufweisen, zusätzlich auch den Transfer nicht veränderter Lipide, wie solche aus Hirsen, Buchweizen, Dinkel, Tapioka, (insbesondere mit den vorstehend angegebenen bevorzugten mittleren Molekülmassen, Transferraten), was zu einer weiteren unerwarteten Verbesserung des Systems durch beschleunigten Transfer nicht veränderter, bzw. regenerierter Lipide führen kann. Diese sind daher auch bevorzugt. Erfindungsgemäß werden in einer weiteren bevorzugten Ausführungsform auch Proteine mit Transferraten für nicht strukturell, insbesondere oxidativ nicht veränderte Lipide, wie sie im Stand der Technik (DE 38 15 473 C1 oder DE 103 24 256 A1) beschrieben sind, weiterhin enthalten sein, welche sodann zu einem weiteren katalytisch aktivierten Transfer nicht veränderter Lipide, vor allem regenerierter Lipide, in die entsprechenden Aggregate führt. Dazu gehören bevorzugt Proteine aus Hafer, Weizen, Mais (mittleres MW 3800 bis 7700, insbesondere 5800 bis 7700), Milch (mittleres MW z. B. 2700 bis 6800), Meeresfrüchten (mittleres MW 11500 bis 23000) oder Mischungen hiervon, wie insbesondere in DE A1 103 24 256 beschrieben. Insgesamt liegt also ein Recycling vor. Die Transferaktivität derartiger Proteine für nicht strukturell, insbesondere oxidativ nicht veränderte Lipide kann wie bekannt ermittelt werden, siehe DE 103 24 256 A1.

### 2. Lipiddepot

Zur Bereitstellung eines erfindungsgemäßen Lipiddepots werden strukturell unveränderte Lipide eingesetzt.

Hierzu gehören insbesondere Lecithine wie Soja- Eilecithin, Sphingolipide, Phospholipide, welche auch Vesikelbildner darstellen. Mit letzteren ist die Ausbildung von uni - oder multilamellaren Liposomen entweder per se z. B. in Gegenwart geeigneter tensidischer Zusätze / Wasser oder auch extern auf bekannte Weise wie Hochdruck- Homogenisation, Ultraschall- Verfahren, Extrusionsverfahren, Detergenzdialyse möglich. Mizellare Emulsionen werden aus polaren Lipiden erhalten.

Als Lipiddepot können auch weitere übliche Fette, Öle, Wachse eingesetzt werden, welche zur Aufnahme des nicht transferierbaren Wirkstoffs beitragen. Hierzu gehören vor allem Kohlenwasserstoffe wie Squalen, Squalan, insbesondere auch flüssige Paraffine, Isoparaffine, Dioctylcyclohexane, Isodecan, Isohexadecane.

Auch natürliche oder synthetische Fette, Öle, Wachse, Polysiloxanverbindungen, Triglyceride oder Mono- / Diglyceride von C₁₂₋₂₂- Fettsäuren, C₈₋₂₂-Fettalkohole wie Oleylalkohol, Octyldodecanol, Cetyl/stearylalkohol; C₁₂₋₂₂ - Fettsäurepartialester von mehrwertigen C₂₋₆- Alkoholen wie insbesondere Monoglyceride, oder Diglyceride z. B. Glycerolmono- / Distearat, Mischungen hiervon; Polyol C₁₂₋₂₂ - Fettsäureester wie PEG-7- Glyceryl Cocoate, Propylen Glykol Dicaprylate /di-caprate,Gemische wie Hexyldecanol und Hexyldecyl Laurat, C₁₂₋₂₂-Fett-säure-partialester von C₂₋₆-Alkoholen sind geeignet. Auch Fettsäureester, z.B. C₂₋₁₈-Alkoholfettsäureester wie Isopropylfettsäureester (Palmitat, Myristat, Isostearat, Oleat), Decyl Oleate, Hexyl Laurate, C 12 - 15 Alkyl Benzoate, Dicaprylyl Carbonate sowie verzweigte Fettsäureester wie Cetearyl Octanoate, Di-n-Butyl- adipate sind geeignet. Weiterhin verwendbar sind C₈₋₂₂- Fettalkoholether wie Dicaprylyl Ether oder Fettalkoholester wie C₁₂₋₁₃- Alkyl Lactate, insbesondere Glyceride (C₁₂₋₂₂ Fettsäure-Glyceride) wie Triglyceride, insbesondere mittelkettige (Neutralöle) wie Caprylic/Capric Triglyceride sowie deren Polyolester wie Propylene Glycol Dicaprylatel Dicaprate.

Zu natürlichen Fetten / Ölen Wachsen gehören z. B. Sonnenblumen-, Soja-, Pfirsichkem-, Aprikosenkem-, Traubenkem-, Ricinus-, Oliven- Erdnuß-, Mandel-, Nerz-, Weizenkeim-, Avocadoöl, Shea Butter oder Illipé Butter, natürliche flüssige Wachse, z.B. Jojobaöl oder dessen Substitut OleylErucate, natürliches Bienenwachs und ähnliches.

Synthetische oder halbsynthetische Wachse sind z. B. gebleichtes Bienenwachs, Kester^{®}Wachs K82H (C₂₀₋₄₀- Alkylstearat) oder Lunacera® M (Micro Wax) oder Kohlenwasserstoffwachse wie Lunacera^{®} P (Mineral Wax) sowie hydriertes Rizinusöl (Cutina^{®} HR) oder synthetische Wachse wie Cetylpalmitat (Cutina^{®} CP) oder Myristylmyristat (Crodamol^{®} MM), oder Stearylstearate (Crodamol^{®} SS). Polysiloxanverbindungen sind z. B. Silikonölewachse wie Polydimethylsiloxane (Dimethicone), Cyclomethylsiloxane (Cyclopentasiloxane), Phenylmethylpolysiloxane wie Phenyl Dimethicone oder Alkyl-Polymethyl-siloxan-Copolymere wie Cetyl Dimethicone, Stearyl Dimethicone , Dialkoxydimethylpolysiloxane wie Stearoxy Dimethicone, Behenoxy Dimethicone, welche insbesondere in Kombination mit anderen oben genannten Lipiden eingesetzt werden können. Die Lipide werden wie erwähnt insbesondere als liposomale Phase oder auch, wenn z. B. die übrigen Lipidsubstanzen alleine oder in Kombination mit Vesikelbildnem eingesetzt werden, als Emulsionen, insbesondere mizellare Emulsionen eingesetzt. Dazu werden geeignete Additive wie nachfolgend genannt, insbesondere Wasser und oberflächenaktive Substanzen, eingesetzt und die Mischung aus Lipid, Additiv in geeigneten Vorrichtungen z. B. durch Rühren und / oder Homogenisieren zu den gewünschten Emulsionen umgesetzt. Neben den vesikelbildenden Lipiden, insbesondere Phospholipiden, Lecithinen wie Soja- Ei- Lecithin, Phosphatidylcholin, Phosphatidylserin, Phosphatidylethanolamin, Phophatidylglycerol, Phosphatidylinositol, Ganglioside, Cerebrosiden, Cholesterin und Ceramiden, wie z.B. Ceramid-2, -3, -6 sowie Sphingolipiden sind insbesondere natürliche Öle, Fette und Wachse wie genannt, Triglyceride , C₈₋₂₂-Fettalkohole; C₁₂₋₂₂ - Fettsäurepartialester von mehrwertigen C₂₋₆- Alkoholen als Lipiddepot geeignet.

Es können auch einfache stabile Emulsionen wie z. B. O/W oder W/O Emulsionen unter Einsatz geeigneter Additive wie nachfolgend genannt hergestellt werden. Bevorzugt sind mizellare Emulsionen aus polaren Lipiden, sowie Lipiddepots aus Phospholipiden, Lecithinen wie Soja- Ei- Lecithin, Phosphatidylcholin, Phosphatidylserin, Phosphatidylethanolamin, Phophatidylglycerol, Phosphatidylserin, Phosphatidylinositol, Ganglioside, Cerebrosiden, Cholesterin und Ceramiden, wie z.B. Ceramid-2, -3, -6 sowie Sphingolipiden.

In einer anderen geeigneten Ausführungsform können lamellare Lipiddepots wie beschrieben auch mit natürlichen und / oder synthetischen Lipiden, insbesondere Triglyceriden, gesättigten, ungesättigten, partialgesättigten C₆₋₂₄- Fettalkoholen, C₈₋₃₀Fettsäureestern, vor allem den natürlichen Vertretern der genanten Gruppen, insbesondere natürlichen Ölen und Wachsen wie Sonnenblumenöl, Olivenöl, Pfirsichkernöl, Weizenkernöl, Orangenöl, Mandel- Raps- Soja-, Kokosnussöl, Pflanzenwachsen, Fruchtwachsen, Bienenwachs, in Kombination eingesetzt werden.

Die Lipiddepotmenge richtet sich nach der jeweils gewünschten Anwendungsform und beträgt bevorzugt 1 bis 70% Gew. %, insbesondere 3 bis 60 Gew.% und vor allem 8 bis 57 Gew.%. Wenn Lotionen, Sprays, oder gelförmige Produkte gewünscht sind, beträgt die Lipidmenge bevorzugt 5 bis 45 Gew%. Insbesondere für balneologische Zwecke, z. B. als Badezusatz können zwischen 25 und 70 Gew.% Lipide, als Duschzusatz bevorzugt 5 bis 35 Gew.% Lipide vorhanden sein. Bei Formulierungen in Form einer (z. B.W/O) FettCreme können insbesondere 5 bis 60 Gew. Lipiddepot enthalten sein.

### 3. Nicht transferierbare Wirkstoffe

Zu den nicht transferierbaren Wirkstoffen zählen insbesondere solche, die durch Wechselwirkung mit strukturell veränderten Lipiden diese in deren Grundstruktur rückführen können. Dazu gehören insbesondere folgende Substanzen: höhermolekulare Enzymproteine wie z. B. Peroxidasen, Kofaktoren wie Gluthathion und analoge Sulfhydrylverbindungen, Antioxidantien wie Ubichinone, pflanzliche phenolische Verbindungen wie z. B. Cystus-Tee- Extrakt, Apfelquercetin, Redoxsysteme wie Erythorbinsäure oder Mischungen hiervon. Besonders geeignet sind diese Wirkstoffe, vor allem Peroxidasen, Kofaktoren wie Gluthathion, Cystus-Tee- Extrakt, Apfelquercetin, Erythorbinsäure, zum Recycling von oxidierten Lipiden.

Weiterhin können auch wasserlösliche bzw. mindestens wasserdispergierbare nichttransferierbare Wirkstoffe, ausgewählt aus Ascorbinsäure, α-Liponsäure, NADH, NADPH, Grüntee- Extrakt, oder Mischungen hiervon eingesetzt werden. Geeignet sind auch wasserlösliche Extrakte aus Heidelbeeren, Holunderbeeren, oder auch aus Trauben, Erdbeeren, oder Gemische hiervon.

Ebenfalls möglich sind Rutin, Ferulasäure.

Besonders bevorzugt sind Peroxidasen, Gluthathion, analoge Sulfhydrylverbindungen, Ubichinone.

Diese Wirkstoffe sind insbesondere wasserdispergierbar bis wasserlöslich und werden herstellungsgemäß in das Lipiddepot auf an sich bekannte Weise inkorporiert. Dazu wird zunächst die wässrige Phase bereitet, hier der oder die gewünschten Recycling Wirkstoff(e) eingearbeitet, sodann die Lipiddepotphase hergestellt und beide Phasen anschließend unter Einsatz geeigneter bekannter Apparaturen und ggf. auch weiterer Additive wie Kolloide, oberflächenaktive Stoffe, Konsistenzregulatoren etc, miteinander vermischt. Die nicht transferierbaren Wirkstoffe liegen in anwendungsbekannten Mengen wie 0,01 bis 9 Gew. %, insbesondere 0,1 bis 7% und bevorzugt 0,01 bis 5 Gew. % je nach System vor.

### 4. Zusatzstoffe

Die Art der Zusatzstoffe richtet sich nach der gewünschten Applikationsform und umfasst insbesondere Wasser, sowie Additive.

Die Additive werden vor allem ausgewählt aus Grenzflächenaktiven Stoffen, Kolloiden, Pflegestoffen, Konsistenzreglem, Zusatzwirkstoffen, Konservierungsmitteln, Parfümstoffen, Farbstoffen oder Mischungen hiervon. Grenzflächenaktive Stoffe sind Emulgier oder Dispersionsmittel und können in Mengen von 0,1 bis 20 Gew.% enthalten sein, bevorzugt 0,1 bis 10 Gew.%, insbesondere 1 bis 7 Gew.%. Dazu gehören W/O- Dispersionsmittel wie bevorzugt Sorbitanderivate (Sorbitanoleat, Sorbitanstearat), ethoxylierte Fettsäuren /Alkohole/Ester wie PEG2/4Stearat, Ceteareth 3, (Poly)glycerylester (z.B.Polygly-ceryl-3-stearat), Glycerinester wie Glyceryl Ricinoleate, Polymere wie Polyoxypropropylen-polyoxyethylen-Blockpolymere, Polysiloxan-Copolymere wie Polysiloxan-Polyether-Copolymere, insbesondere Polysiloxan-Polyalkyl-Polyether-Copolymere wie Cetyl Dimethicone Copolyol, mehrwertige Salze wie Magnesium-, Aluminium-, oder Zinkstearat, Triglyceride wie ethoxylierte C₈₋₂₂Fettalkohole- / C₁₂₋₂₂Fettsäuren, C₁₂₋₂₂Fettsäuremono- und Diester von Anlagerungsprodukten von Ethylenoxid an C₃-C₆Polyole, ethoxylierte Alkylglykoside, (Poly)glycerylderivate, Polyolester, Pentaerythrit- Derivate, Alkylphenole, oder Mischungen hiervon und hierunter ganz besonders Abil® EM 90, Arlacel ®582 und Magnesiumstearat oder Mischungen hiervon. Es können hier auch Co- Emulgatoren vorhanden sein, wie Stearyl- Cetearylalkohol, Stearinsäure, wie z.B. Arlatone®T(V). Ferner gehören zu grenzflächenaktiven Stoffen O/W-Emulgatoren wie insbesondere polyoxyethylierte Produkte wie solche vom Magrogol- Typ (PEG Fettsäuren/ Alkohole/ Fettsäureester mit einer Kettenlänge von 6 bis 22 C- Atomen im Fettsäure/esterteil und Ethoxylierungsgraden von 5 bis 30), nichtionische und ionische Phosphate, ionische einwertige Salze, Sterol- Derivate, Rizinusöl-Derivate, Siloxane oder Gemische hiervon oder Mischungen mit Co- Emulgatoren hiervon. Als O/W- Emulgator eignen sich insbesondere polyoxyethylierte Produkte, nichtionische und ionische Phosphate, ionische einwertige Salze, (Poly)glycerylester, Sterol- Derivate, Rizinusöl-Derivate, Siloxane oder Gemische hiervon oder Mischungen mit Co- Emulgatoren hiervon. Insbesondere sind hier Tego Care® 450, Eumulgin ®B1 oder Mischungen hiervon und/oder mit Co-Emulgatoren geeignet. Analog sind als W/O- Emulgatoren insbesondere bevorzugt Sorbitan- Derivate, Polyethoxylierte Fettsäuren/Alkohole/ Ester/, Triglyceride, (Poly)glycerylderivate, Polyolester, Glucose-Derivate, Pentaerythrit-Derivate, Alkylphenole, (Block)Polymere, Fettsäuresalze, Siloxane oder Mischungen hiervon und hierunter ganz besonders Abil® EM 90, Arlacel ®582 und Magnesiumstearat oder Mischungen hiervon.

Es können hier auch Co- Emulgatoren vorhanden sein, wie z.B. Arlatone®T(V). Je nach Ethoxylierungsgrad und / oder Alkylkettenlänge werden demnach entweder O/W oder W/O Dispergiermittel erhalten, wobei ein höherer Ethoxylierungsgrad und / oder kurze Kettenlänge zu O/W Produkten und umgekehrt zu W/O Produkten führen. Dies ist dem Fachmann bekannt und er kann z. B. anhand des entsprechenden HLB- Wertes (2 bis 7 = W/O, 8-18=O/W_{,} Grenzbereich 9 bis 13 wasserdispergierbar) geeignete Produkte auswählen. Somit können die gewünschten Emulsionen z. B. durch Kombination geeigneter Produkte erhalten werden.

Weiterhin sind auch (Co)- Tenside als grenzflächenaktive Substanzen möglich, insbesondere anionische und nichtionische Tenside, welche auch besonders in Anwendungen mit zusätzlicher Reinigungsfunktion vorhanden sein können. Dazu gehören vor allem als nicht ionische Tenside alkoxylierte Fettsäureester z. B. der Formel R1CO(OCH2CHR2)ₓCR3 mit R1CO: linearer verzeigter, gesättigter und / oder ungesättigter C6-22Acylrest, R2 =H oder Methyl, R3 =C1-4Alkyl, x=1-20 und Ähnliches (z. B. Glyceride).

Anionische Tenside sind Glutamate, z.B. Sodium Cocoyl Glutamate (Hostapon^{®} CCG) oder auch solche, ausgewählt aus Alkylsarcosinaten, Alkylsulfaten, Alkylethersulfaten, Alkylsulfosuccinaten, Alkylsulfosuccinnamaten, Isethionaten, bzw. deren Alkalisalze oder Mischungen hiervon geeignet. Diese weisen insbesondere 8 bis 22, bevorzugt 8 bis 16 Kohlenstoffe in der Alkylkette auf. Insbesondere sind hier Alkylethersulfate bevorzugt, die sich von Fettalkoholen mit 12 bis 18 Kohlenstoffatomen und einem Ethoxylierungsgrad von 2 bis 6 ableiten, wie z.B. Lauryl/Myristylethersulfat, Na-Salz, Ammoniumlaurylethersulfat oder Monoisopropanolammoniumlaurylethersulfat bzw. Alkylsulfate, z.B. Natriumlaurylsulfat, Ammoniumlaurylsulfat oder Monoisopropanol-ammoniumlaurylsulfat. Ganz besonders bevorzugt sind Alkylsarcosinate wie Sodium Lauroyl, Cocoyl oder Oleyl Sarcosinate, Bemsteinsäurederivate wie Alkylsulfosuccinate und Alkylsulfosuccinamate mit einem Alkylrest von 8 bis 22 Kohlenstoffatomen Mischungen hiervon. Vor allem sind die genannten Alkylsarcosinate bevorzugt.

Emulsionen, insbesondere Emulsionsgele können bevorzugt auch durch Einsatz von Kolloiden gewonnen werden.

Kolloide (z.B. bevorzugt 0,01 bis 8%) sind insbesondere hoch- bzw. höhermolekulare Stoffe natürlichen oder (halb)synthetischen Ursprungs oder Kombinationen hiervon wie hochmolekulare pflanzliche Proteine mit einer Molekülmasse von 100 000 und mehr D, höhermolekulare tierische Proteine wie Caseinate z. B. mit einer mittleren Molekülmasse von 18000 und mehr, Gelatine oder synthetische Polymere wie Acrylatpolymere mit hohem Molekulargewicht (0,2 - 3 Mio.) sowie deren Copolymere, insbesondere nach Neutralisierung durch Alkali. Bevorzugt sind hier die unter dem INCl- Namen Carbomer bekannten Polyacrylat - Carbopol^{®}-Typen, z.B. Carbopol^{®} 910, 934, 940, 941, 954, 980, 981, 2984, 5984 oder Carbopol^{®} ETD 2001, 2050 oder Synthaten^{®} K, L, M oder die bereits neutralisierten Carbomere wie PNC^{®} 400, 410, 430 (INCl: Sodium Carbomer). Es können auch Acrylat- Copolymere eingesetzt werden, z.B. Arylates/ C10-30 Alkyl Acrylate Crosspolymer, bekannt als Carbopol^{®} 1342, 1382, ETD 2020, Pemulen^{®} TR-1, TR-2.

Ferner geeignete Kolloide sind Acrylamide sowie Polysaccharide wie Keltrol^{®} (Xanthan Gum), Cellulose-Derivate wie Hydroxypropyl Methyl Cellulose (Methocel^{®} J12-MS) oder Ethylcellulose oder Silikate wie Magnesium Aluminium Silikat (Veegum^{®} HV) mit jeweils hohem Molekulargewicht, insbesondere von mehr als 50000.

Bevorzugte Acrylamide sind Polyacrylamid, z.B. Flocare^{®} T 920 GC, ggf. Polyacrylamidhaltige Gemische wie Sepigel^{®} 305 (Polyacrylamide, C13-14 Isoparaffin, Laureth-7), Sepigel^{®} 501 (Acrylamides Copolymer, Mineral Oil, C13-14 Isoparaffin, Polysorbate 85), Sepigel® 502 (C13-14 Isoparaffin, Isostearyl Isostearate, Sodium Polyacrylate, Polyacrylamide, Polysorbat 20), Creagel^{®} EZ DC (Polyacrylamide, Polydecene, Dimethicone Copolyol), Creagel^{®} EZ 5 (Polyacrylamide, Polydecene, Laureth-5).

Kolloide werden bevorzugt mit Co- Emulgatoren wie den nachfolgend unter Konsistenzreglern genannten Fettalkoholen eingesetzt.

Pflegestoffe sind z. B. (z.B. bevorzugt 0,01 bis 20%, insbesondere 0,01 bis 10%) Phytosterole (im wesentlichen Gemische aus β-Sitosterol, Campesterol und Stigmasterol) wie solche aus Rapsöl (Generol^{®} R), Silikon -(Co)Polymere, z.B. Dow Corning^{®} HMW 2220 (Divinyldimethicone/ Dimethicone Copolymer, C12-13 Pareth-3/C12-13 Pareth-23). Weitere Pflegestoffe sind Feuchthaltemittel wie Glycerin, Propylenglykol oder Polyethylenglykole, Propylenglykol, Butylenglykol, Sorbitol oder Polymere, z.B. Polyquaternium-Typen wie Polyquaternium -39 (Merquat^{®} plus 3330), Aminosäuren, Harnstoff, Polysaccharide wie z.B. Fucogel^{®} 1000 (Biosaccharide Gum-1), Glucosaminoglykane, z.B. Hyaluronsäure oder sulfatierte Glucosaminoglykane wie Chondroitinsutfat, Dermatansulfat, Keratansulfat, Heparansulfat, insbesondere deren Na-Salze oder Heparin-Na , Glukane, z.B. β-Glukan, z.B. Hafer β-Glukan (Drago-β-Glucan^{®}, Mannane wie Konjac Mannane, oder handelsübliche Feuchthaltemittel wie z.B. Hydractin^{®} (Glycerin, Aqua, Disodium Adenosine Triphosphate, Algin, Carica Papaya) oder Aquaderm^{®} (Sodium PCA, Sodium Lactate, Fructose, Glycine, Niacinamid, Urea, Inositol), Salze, z.B. Natriumlactat, DL-2-Pyrrolidon-5-Carbonsäure, Na-Salz. Bevorzugt sind Feuchthalter wie Polyethylen-/ Propylenglykol oder Glycerin in Mengen von z.B. 0,5- 10%, insbesondere 2- 5%, sowie Polysaccharid-Verbindungen wie Fucogel^{®} 1000, und/oder Hyaluronsäure, Na-Salz. Konsistenzregler sind z. B. Komplexbildner wie Trilon^{®}BD (EDTA, Na-Salz), Mittel zur pH- Einstellung, z.B. Zitronensäure, Natronlauge, Lösungsmittel wie Propylenglykol oder Alkohole, Stärke, bzw. Stärke-Derivate Stärke wie Reis-, Weizen-, Mais- und Kartoffelstärke, hydrophobisch modifizierte Stärken wie Dry Flo^{®} AF (Corn Starch Modified), Aluminium Starch Octenylsuccinate (Dry Flo^{®}. PC, Fluidamid^{®} DF 12), Hydroxypropyl Starch Phosphate Ester (Structure^{®} XL) oder dessen Gemische wie Natrasorb® HFB (Aluminium Starch Octenylsuccinate, Acrylates Copolymer, Magnesium Carbonate), ASO/MM3^{®} (Aluminium Starch Octenylsuccinate, Magnesium Myristate), Dry Flo^{®} Elite LL (Aluminium Starch Octenylsuccinate, Lauroyl Lysine), Facemat^{®} (Aluminium Starch Octenylsuccinate, Mica, Zea Mays (Corn) Starch, Silica, Titanium Dioxide, Zink Oxide), Mischungen genannter Substanzen hiervon.

Weitere geeignete Konsistenzregler sind z. B. Fettalkohole, wie Stearyl Alcohol (Lanette^{®} 18), Cetyl Alcohol (Lanette^{®} 16), Myristyl Alcohol (Lanette^{®} 14) oder Cetearyl Alcohol (Lanette^{®} O). Ferner geeignet sind Glycerylester wie Glycerylstearat, insbesondere Glycerinmonostearat oder Glycerindistearat oder Gemische hiervon, z.B. Tegin^{®} M. Diese können ggf. auch als Co- Emulgatoren aufgefasst werden.

Die erfindungsgemäßen Zusammensetzungen sind insbesondere Haut-neutral und Haut- bzw. Schleimhaut-freundlich und weisen insofern bevorzugt einen pH-Wert von ≤ 7, auf.

Daneben können gegebenenfalls ein oder mehrere Konservierungsmittel (bevorzugt z.B. je 0,01 bis 5 %) vorhanden sein. Geeignete Konservierungsmittel sind beispielsweise lodopropynylbutylcarbamat, DMDM Hydantoin, Phenoxyethanol und weitere gebräuchliche Konservierungsstoffe, wie z.B. Sorbin- und Dehydracetsäure und deren Salze, Methyldibromoglutanonitril, etc. oder Kombinationen hiervon, oder andere Säuren wie Benzoe- oder Salicylsäure, oder Benzylalkohol oder Ester wie p-Hydroxy- benzoesäureester, z.B. Methyl-, Ethyl-, Propyl-, Butyl-, Iso-Butylparaben, bevorzugt Methyl-oder Propylparaben oder Mischungen hiervon oder Climbazol oder geeignete Kombinationen der genannten Stoffe.

Geeignete Parfümstoffe (z.B. bevorzugt 0,01 bis 5%) sind z.B. die unter Wirkstoffen genannten ätherischen Öle oder handelsübliche Parfümmischungen. Als Farbstoffe (z.B. bevorzugt 0,01 bis 2%) werden vorzugsweise die für gattungsgemäße Produkte bekannten Komponenten gewählt wie z.B. Patentblau, Amidoblau, Orange RGL, Cochenillerot, Chinolingelb.

Zusatzwirkstoffe (z. B. 0,01 bis 10%) sind insbesondere Vitamine, Extrakte aus Pflanzen, diesen Extrakten entsprechende synthetisch hergestellte Substanzen und analoge Derivate hiervon, sowie Haut beeinflussende Wirkstoffe und Mischungen hiervon.

Extrakte aus Pflanzen sind beispielsweise solche aus Ylang Ylang, Gingko, Kiefemnadel, Zypresse, Birkenblätterextrakt, Aloe-Vera-Extrakt, Ringelblumen-, Hibiskus-, Klettenwurzel-Hamamelis-, Wassernabelkraut-, Algen-, Quitten-, Wasserlilien-, Zimtextrakt, Extrakte aus Thymian, Minze, Limetten, Orangen, Grapefruit, Mandarine, Wacholder, Baldrian, Zitronenmelisse, Eukalyptus, Thymian, Palmarosa, Rosmarin, Lavendel, Rosenholz, Lemongras, Wildrose, Fichtennadel, Kiefernnadel, Ingwer-, Johannisbeer-, Lindenblüten-, Ringelblumen-Magnolien-, Ananas-, Guave-, Echinacea-, Efeublätterextrakt oder Mischungen hiervon.

Diese Extrakte können hergestellt werden z.B. durch Wasserdampfdestillation. Hierdurch werden z.B. ätherische Öle aus den genannten Pflanzen erhalten, welche besonders bevorzugt sind.

Die Extrakte können auch auf andere bekannte Weise durch z.B. Lösungsmittelextraktion (mit Alkoholen, Triglyceriden oder Kohlenwasserstoffen, Wasser, Gemischen hiervon) erhalten und als solche dann eingesetzt werden. Analoge synthetisch hergestellte Substanzen sind beispielsweise Terpene und Terpenoide wie Campher, Menthol, Cineol oder Mischungen hiervon.

Als Vitamine eignen sich z. B. Vitamin A, E, oder andere bzw. geeignete Derivate hiervon wie Ester z.B. Palmitat, Acetat oder Phosphat.

Weiter geeignet sind Mineralstoffe und Spurenelemente wie Kupfer, Zink, bzw. deren Derivate wie Zincidone^{®} (Zink PCA), Zinkglukonat oder Kupfergluconat. Weiterhin können adstringierende und sebumregulierende Substanzen eingesetzt werden wie Acnacidol^{®} 101 (Propylene Glykol, Hydroxydecanoic Acid), Asebiol^{®} BT (Hydrolyzed Yeast Protein, Pyridoxine, Niacinamide, Glycerin, Panthenol, Propylene Glycol, Allatoin, Biotin), Lipacide^{®} C8C0 (Caproyl Collagen Aminoacids), Sebosoft^{®} (Glycerin, Aqua, PEG-8, Caprylyl Glycol, Sebacic Acid, Sodium Polyacrylate), Sepi Control A5 (Capryloylglycine, Methylglycine, Cinnamonum zeylanicum).

Darüber hinaus sind als Zusatzstoffe durchblutungsfördernde Stoffe, z. B. Nikotinsäurederivate wie Methyl- oder Tocopherylnikotinat, Alpha- und Betahydroxysäuren und deren Derivate, z. B. Glykol-, Äpfel-, Zitronen-, Wein-, Milchsäure, Salicylsäure, Isopropylbenzylsalicylate, C12 - 13 Alkyl Lactate (Cosmacol^{®} ELI) oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat, Panthothensäurederivate, z. B. D-Panthenol, Panthenyltriacetat; Allatoin; Bisabolol; Azulene, z. B. Cham-Azulene oder Guaj-Azulen; Phytosphingosine; Triclosan; Chlorhexidin-Derivate und/oder Antischuppenmittel, z. B. Climbazol oder Piroctone Olamine einsetzbar.

Daneben können in den erfindungsgemäßen Zusammensetzungen auch zellschützend wirkende Stoffe wie Coenzym Q 10 als wirksame Zusatzstoffe eingesetzt werden.

Weitere Zusatzwirkstoffe sind UV-Filter wie UVB, UVA und Breitbandfilter der folgenden Art:

UV-B Filter: Zimtsäureester, z.B. Ethylhexyl Methoxycinnamate Isoamyl p-Methoxycinnamate sowie 4-Methylbenzyliden Camphor, Paraaminobenzoesäure und -ester wie N, N-Dimethyl-4-aminobenzoesäure-2-ethylhexylester, Homomenthylsacilylat, OctylSalicylate, Octocrylene Phenylbenzimidazolesulfonic Acid oder Benzylidene Malonate Polysiloxane, UVA + UVB Filter für Breitbandabsorption wie Benzophenone-3 (Neo^{®} Heliopan BB, Eusolex^{®} 4360). UV-A Filter wie Methyl Anthranilate , Butyl Methoxydibenzoylmethane, Methylene Bis-Benzotriazolyl Tetramethylbutylphenol, Bis-Ethylhexyloxyphenol Methoxyphenyltriazin, Disodium Phenyl Dibenzimidazole Tetrasulfonate 2-(4-Diethylamino-2-hydroxybenzoyl)benzoic Acid Hexylester.

Dabei kann je nach beabsichtigtem zusätzlichem Wirkungseffekt eine geeignete Kombination an Wirkstoffen zugesetzt werden.

Besonders bevorzugte Zusatzwirkstoffe sind ätherische Öle oder Extrakte aus Ylang Ylang, Lemongras, Minze, Orangen, Wildrose, Grapefruit, Limette, Gingko, Mandarine, Zypresse, Thymian, Lavendel, Rosmarin, Ingwer, oder Mischungen hiervon.

Ferner werden vor allem auch die genannten Vitamine und/oder Panthenol eingesetzt, insbesondere in Kombination mit einem oder mehreren Pflanzen- Extrakten / ätherischen Ölen.

Besonders erwünschte Zusatzwirkstoffe sind ausgewählt aus Vitaminen, UV-Filtern, Extrakten aus Pflanzen oder auch dermatologischen Wirkstoffen, z. B. Antimykotika wie Naftifin HCl, Terbinafin HCl oder antioxidativen (vor allem in geringen Mengen), antibakteriellen und/oder sebumregulierenden Stoffen.

Die Art und Menge der Additive richtet sich nach der gewünschten Anwendungsform und dem gewünschten Zweck. Es ist bevorzugt, wenn höchstens 40%, insbesondere 0,01 bis 30%, vor allem auch 0,01 bis 25%, bevorzugt 0,1 bis 20% oder 1 bis 18 %, insbesondere 5 bis 15% an Additiven vorhanden sind. Besonders bevorzugt werden als Zusatzstoffe neben Wasser Konsistenzregler, Farbstoffe, Parfümstoffe, Kolloide, grenz(ober)flächenaktive Stoffe, Pflegestoffe, ggf. Konservierungsmittel oder Mischungen hiervon gewählt, wobei Pflegestoffe, Konsistenzregler, Zusatzwirkstoffe besonders bevorzugt werden.

Besonders bevorzugt sind Zusatzstoffe, ausgewählt aus Wasser und 0,01 bis 30%, insbesondere 0,1 bis 20 Gew.% Additiven, welche vor allem ausgewählt sind aus Zusatzwirkstoffen, Pflegestoffen, Farbstoffen, Parfümstoffen, grenzflächenaktiven Stoffen, insbesondere Kolloiden, Co- Tensiden, Konsistenzreglem, Konservierungsstoffen.

Bevorzugte Zusammensetzungen sind dadurch gekennzeichnet, dass 0,01 bis 10 Gew. % eines oder mehrerer Transferproteine b), 1 bis 70 Gew. %, bevorzugt 1 bis 50 Gew.%, insbesondere 1 bis 40 Gew.% des Lipiddepots a), 0,01 bis 10 Gew. % eines oder mehrerer nicht transferierbarer Wirkstoffe c) und d) 1 bis 30 Gew.%, vor allem 1-20 Gew.% Additive, insbesondere ausgewählt aus Zusatzwirkstoffen, Pflegestoffen, Farbstoffen, Parfümstoffen, grenz(ober)flächenaktiven Stoffen wie angegeben, Kolloiden, Konsistenzreglern, Konservierungsstoffen oder Mischungen hiervon sowie Wasser (als Rest) enthalten sind.

In einer weiteren Ausführungsform sind 0,05 bis 5 Gew., insbesondere 0,1 bis 3 Gew.%, Transferprotein b), 1 bis 60 Gew. %, vor allem 5 bis 53 Gew.%, Lipiddepot a), 0,05 bis 5 Gew.%, insbesondere 0,1 bis 5 Gew.% Wirkstoff c), und d) 1 bis 25 Gew.%, bevorzugt 1 bis 20, insbesondere 1 bis 15 Gew.% Additive, ausgewählt aus Zusatzwirkstoffen, Pflegestoffen, Farbstoffen, Parfümstoffen, grenz(ober)flächenaktiven Stoffen wie O/W W/O, CO- Emulgatoren, Co-Tensiden, Kolloiden, Konsistenzreglem, Konservierungsstoffen oder Mischungen hiervon sowie (als Rest) Wasser enthalten.

### 5. Applikationsformen:

Je nach Wassergehalt, der zwischen 5 und 80 Gew. % liegen kann, können folgende (beispielhafte) Applikationsformen erhalten werden (mit jeweils beispielhaft genannten Zusätzen):
Gele (z. B. 50 bis 80 % Wasser) mit Kolloiden, Co- Emulgatoren, und wahlweise Zusatzwirkstoffen, Pflege- Farb-Parfümstoffen, Konsistenzreglern oder Mischungen hiervon;
O/W oder W/O Cremes (z. B. 20 bis 70% Wasser) mit grenzflächenaktiven Stoffen / Kolloiden und wahlweise Zusatzwirkstoffen, Pflege- Farb-Parfümstoffen, Konsistenzreglern oder Mischungen hiervon;
Balsam: (z. B. 10 bis 60% Wasser) : mit grenzflächenaktiven Stoffen/ Kolloiden und wahlweise Zusatzwirkstoffen, Pflege- Farb-Parfümstoffen, Konsistenzreglern oder Mischungen hiervon;
Bade- oder Duschzusatz: (z. B. 30-80% Wasser) mit Kolloiden, Co- Tensiden, Co-Emulgatoren, und wahlweise Zusatzwirkstoffen, Pflege- Farb-Parfümstoffen, Konsistenzreglern oder Mischungen hiervon.

### Funktionsweise des Recyclingsystems

Die erfindungsgemäßen Zusammensetzungen weisen ein Lipiddepot, spezielle Proteine und spezielle Wirkstoffe wie charakterisiert zum katalytischen Transfer bzw. Recycling strukturell veränderter Lipide auf. Die Wirkstoffe werden dabei in das Lipiddepot inkorporiert, aber nicht transferiert. Auf diese Weise bleibt die thermodynamische Triebkraft für den Transfer veränderter Lipide erhalten, so dass sich der Prozess nicht durch Gleichverteilung in den beiden Kompartimenten (Lipiddepot ohne / Lipidaggregat mit strukturell veränderten Lipiden) erschöpft, wie dies bei den im Stand der Technik beschriebenen Systemen der Fall ist. Daher ist es möglich, den Zeitpunkt der Wirkdauer durch die Konzentration des/der nicht transferierten Wirkstoff(e)s zu steuern. Dies kann beispielsweise mit entsprechenden Anwendungsformen erfolgen, wie Nacht-Creme mit höheren Wirkstoffanteilen, Milch (z. B. Reinigungsmilch), Badezusätze mit mittleren Wirkstoffmengen, Duschpräparate mit eher weniger Wirkstoffmenge. Das Recycling der veränderten Lipide erfolgt sodann außerhalb der Lipidaggregatstrukturen, so dass es nicht zu unphysiologischen Anreicherungen von Wirkstoffen kommen kann. Dadurch werden unerwünschte Wirkungen an diesen Strukturen vermieden.

Nach dem Recycling werden erfindungsgemäß gleichzeitig oder auch zusätzlich bei Vorhandensein der entsprechenden Transferproteine gemäß Stand der Technik wie z. B. DE 38 15 473 C1 die recycleten Lipide wieder in die Aggregate zurückgeführt.

Es ergibt sich folgender Nettoeffekt: Unabhängig von der Spezifität des katalytischen Transfers der veränderten Lipide in das erfindungsgemäße Depot werden durch das Recycling außerhalb der Aggregatstrukturen (remote recycling) einseitig die veränderten Lipide aus den Aggregaten entfernt, da sich hinsichtlich der nicht veränderten Lipide durch ihren unveränderten Hin- und Hertransfer kein Nettoeffekt ergibt. Bei gegebener Spezifität des katalytischen Transfers für veränderte Lipide hängt der Nettotransfer für die veränderten Lipide aus den Aggregaten nur noch von der Konzentration der veränderten Lipide ab. Steigt deren Anteil im Lipidaggregat an, so erhöht sich auch deren in das Depot transferierter Anteil.

Das erfindungsgemäße System zum Recycling veränderter, insbesondere oxidativ veränderter Lipide aus Lipidaggregaten weist damit autoregulative Eigenschaften auf, indem die Effizienz des Recycling in dem Maße ansteigt wie die Veränderungen an den Lipidaggregaten zunehmen. Gleichzeitig bleibt wegen des unveränderten Hin- und Rücktransfers nicht veränderter Lipide die physiologische Zusammensetzung der Aggregate weitgehend erhalten, sodass keine unerwünschten Effekte auftreten können. Die erfindungsgemäßen Recycling Transfer- Proteine können daher eingesetzt bzw. verwendet werden zum Transfer /Recycling strukturell, insbesondere oxidativ veränderter Lipide, vor allem durch topische Anwendung, auf die Haut /Schleimhaut oder auch zum Engineering von Biomembranen. Sie können hierzu auch insbesondere verwendet werden in dafür geeigneten Systemen, Zusammensetzungen oder auch zur Herstellung hiervon wie die oben beschriebenen Zusammensetzungen aus den Komponenten a), b), c), d). Derartige Systeme bzw. Zusammensetzungen sind vor allem geeignet für die topische Anwendung, z. B. in Form einer Creme, Gel, Lotion, Spray, Maske, Balsam, Dusch- Badezusatz, Balsam, Milch, zum Transfer / Recycling strukturell, insbesondere oxidativ veränderter Lipide, z. B. der Haut, oder auch der Schleimhaut, z. B. bzgl. der nachfolgend genannten Indikationen, insbesondere bei Haut, Haar.

### Anwendung des Recyclingsystems

Je nach beabsichtigtem Zweck können die erfindungsgemäßen Zusammensetzungen auch durch Wahl geeigneter Zusatzstoffe, eingesetzt werden insbesondere zur kosmetischen, kosmetisch- balneologischen oder auch zur dermatologischen /therapeutischen Anwendung auf Haut, Haar oder Schleimhaut. Die Zusammensetzungen können insofern als Creme, Lotion, Milch, Gel, Gelemulsionen, Milch, Balsam, Spray, als Dusch- Badezusatz, als (ggf. verschäumbare) Maske vorliegen und eingesetzt werden als solche zur Lipidregenerierenden Pflege, Reinigung bei kosmetischen oder kosmetischbalneologischen Anwendungen. Dabei erfolgt die Verbesserung von Erscheinungsbildern gereizter, gealterter, insbesondere Licht geschädigter, trockener, empfindlicher Haut, Haare, d. h. bei funktionell/ strukturell , vor allem oxidativ veränderter Haut / Schleimhaut. Besonders bevorzugt ist die Anwendung in Form einer Creme oder Gelcreme sowie eines Dusch- und oder Badezusatzes oder auch als Maske. Mittels der erfindungsgemäßen Kombination können die eingesetzten Transferproteine ihre Wirkung als Vehikel für veränderte lipophile / amphiphile Komponenten entfalten. Durch den Transfer von veränderten Lipiden, das externe Recycling und den Rücktransport bleiben unerwünschte Substanzen nicht in den Lipidstrukturen zurück. Somit kann eine insbesondere kosmetische Regenerierung oxidativ veränderter Lipide wie beschrieben durch Applikation einer erfindungsgemäßen Zusammensetzung auf die Oberfläche vor allem auf die Haut von Menschen, erfolgen. Durch Wahl geeigneter Zusatzwirkstoffe können weitere Effekte erzielt werden. Die Anwendung kann auch bei dermatologisch / pharmazeutischen Anwendungen erfolgen. Hier sind vor allem entzündliche Zustände, Reizzustände der Haut z. B. infolge Allergie, oder Hautverbrennungen, bei Reizzuständen der Atemwege z. B. infolge Allergie, oder Irritationen mögliche Anwendungsgebiete. Mit den erfindungsgemäßen Zusammensetzungen können bei topischer externer Anwendung insofern veränderte, vor allem oxidativ veränderte Lipidaggregatstrukturen, vor allem von alternder, geschädigter, insbesondere Licht geschädigter, trockener, empfindlicher, entzündlicher Haut von Säugern, insbesondere Menschen, beeinflusst, regeneriert werden.

### Herstellung der Produkte

Die erfindungsgemäßen Zusammensetzungen werden hergestellt, indem man die Lipidphase bereitet durch Mischen, ggf. Erwärmen des/der gewünschten Lipide, ggf. auch durch separate Liposomenherstellung, falls gewünscht, wie beschrieben. Dort können darin lösliche Additive inkorporiert werden. Wasserlösliche bzw. wasserdispergierbare Bestandteile wie Additive, Wirkstoffe der Gruppe c) sowie Protein(e) der Gruppe b) werden in Wasser in der gewünschten Konzentration eingearbeitet, und beide Phasen bei geeigneten Temperaturen wie z. B. 20 bis 80° C, bevorzugt 30 bis 60° C bis Raumtemperatur so durchmischt, dass eine stabile Zusammensetzung in Form einer Emulsion/ Gel / Dispersion, etc erhalten wird. Nach dem Abkühlen können ggf. temperaturlabile Substanzen unter Rühren hinzufügt werden. Die Umsetzung erfolgt dabei derart, dass einerseits eine hohe Dispersität der kohärenten Phase erreicht wird, so dass die nicht transferierbaren Wirkstoffe im Depot vorliegen und andererseits die Transferproteine in ihrer Integrität erhalten bleiben.

### Beispiele

Die Erfindung wird anhand der nachfolgenden Beispiele näher erläutert (Mengenangaben beziehen sich auf Gewichtsprozent soweit nicht anders angegeben):

### Beispiel 1 :Zusammensetzung auf W/O Basis

| | |
|---|---|
| Fettalkoholether (Cetiol® OE) | 5,00 |
| Aprikosenkernöl | 5,00 |
| Bienenwachs | 5,00 |
| Nachtkerzenöl | 2,00 |
| Oxynex 2004 | 0,05 |
| Propylenglykol | 4,00 |
| D- Panthenol | 5,50 |
| Gersteproteinhydrolysat | 0,80 |
| (mittleres MW: ca. 5500) | |
| Gluthathion | 1,50 |
| Sorbitan Isostearat (Arlacel®582) | 7,00 |
| PEG40Sorbitanperoleat (Arlatone TV) | 1,50 |
| Magnesiumsterat | 2,00 |
| Farbstoff (Pigmentpaste) | 2,00 |
| Parfümöl (Deliana) | 0,50 |
| Aqua | ad 100 |

### Beispiel 2 : Zusammensetzung auf O/W Basis

| | |
|---|---|
| Sojalecithin (Phosal SA 50) | 2,00 |
| Jojobaöl | 1,00 |
| Bienenwachs | 5,00 |
| Nachtkerzenöl | 2,00 |
| Micro Wachs (Lunacera® M) | 2,50 |
| Fettalkoholether (Cosmacol®ELI) | 3,00 |
| Hydrolysiertes Milchprotein | 0,50 |
| (mittleres MW 3000) | |
| Algenprotein (mittleres MW ca.18000) | 0,50 |
| Natriumchlorid | 0,50 |
| Polyglyceryl-3methyl Glucose- | 3,00 |
| Distearat (Tego Care®450, O/W) | |
| Carbopol ETD 2020 | 0,25 |
| Hydriertes Rizinusöl (Cutina® HR) | 0,40 |
| Propylenglykol | 4,00 |
| Ringelblumenextrakt | 2,00 |
| Parfümöl Deliana | 0,50 |
| Cystus-Tee-Extrakt | 2,00 |
| Aqua | ad 100 |

### Beispiel 3 O/W- Gel -Emulsion

| | |
|---|---|
| Avocadoöl | 3,0 |
| Stearyl Alcohol | 2,0 |
| Glyceryl Stearat | 2,0 |
| Isopropyl Stearat | 6,0 |
| Dicaprylyl Ether | 3,0 |
| Squalane | 5,0 |
| Phosphatidylcholin | 1,0 |
| Hydrolysiertes Hirseprotein (mittleres MW ca. 4500) | 0,8 |
| Hydrolysiertes Milchprotein (mittleres MW ca. 3500) | 0,6 |
| Butylene Glykol | 2,0 |
| Disodium EDTA | 0,1 |
| Natirumcaseinat (MW ca. 20000) | 3,0 |
| Cyclomethicone | 1,0 |
| Konservierungsstoffe | q.s. |
| Parfüm | 2,0 |
| Apfelquercetin | 0,4 |
| Aqua | ad 100 |

### Beispiel 4 : O / W Reinigungsemulsion

| | |
|---|---|
| Mandelöl | 2,0 |
| Cetyl Alcohol | 2,0 |
| C12-15 Alkyl Benzoate | 3,0 |
| Squalane | 1,0 |
| Cetearyl Isononanoate | 2,0 |
| Algenprotein (mittleres MW 15000) | 1,5 |
| Acrylates /C10-30 Alkyl Acrylate | 0,2 |
| Crosspolymer | |
| Hydroxypropylethylcellulose | 0,2 |
| Glycerin | 3,0 |
| Disodium EDTA | 0,1 |
| Natriumlaurylsarcosinat | 2,0 |
| Triethanolamine | 0,25 |
| Konservierungsstoff(e) | q.s. |
| Parfüm | 0,04 |
| α-Liponsäure | 1,00 |
| Aqua | ad 100 |

### Beispiel 5: Badezusatz

| | |
|---|---|
| Sojaöl | 10,00 |
| Jojobaöl | 5,00 |
| Isopropylmyristat | 10,00 |
| Lecithin | 5,00 |
| Hydrolysiertes Dinkelprotein | 1,20 |
| (mittleres MW 4500-5000) | |
| Laureth 4 | 7,00 |
| Polyoxyethylen(7)Glycerylacetat | 5,00 |
| Panthenol | 3,00 |
| Eukaptyptusöl | 2,50 |
| Parfümöl | 0,80 |
| Heidelbeer-Extrakt | 3,00 |
| Aqua | ad 100 |

### Beispiel 6: Duschzusatz

| | |
|---|---|
| Olivenöl | 3,00 |
| Mandelöl | 1,50 |
| Sphingosin | 1,50 |
| Hydrolysiertes Milchprotein | 1,00 |
| (MW 6000) Seidenprotein (MW 12000) | 0,50 |
| Xanthan Gum | 0,50 |
| Hydroxypropylmethylcellulose | 1,50 |
| Cystus- Tee- Extrakt | 1,50 |
| Aqua | ad 100 |

## Patentansprüche

1. Zusammensetzungen enthaltend:
a) ein Depot aus einem oder mehreren strukturell / funktionell nicht veränderten Lipiden, ausgewählt aus natürlichen oder synthetischen Fetten, Ölen, Wachsen, Fettalkoholen, Fettsäureestern, Fettsäurepartialestem, Glyceriden, Silikonölen, Silikonwachsen, Kohlenwässerstoffen. Lecithinen, Sphingolipiden, Cholesterinen, Phospholipiden, Gangliosiden, Cerebrosiden, Ceramiden oder Mischungen hiervon;
b) ein oder mehrere Recycling-Transferproteine mit Transferraten für strukturell / funktionell veränderte, insbesondere oxidierte Lipide, die eine Transferrate von 600 bis 8000ng/min/mg Protein und eine mittlere Molekülmasse von 3500 bis 8000 D aufweisen und ausgewählt sind aus Proteinen aus Buchweizen, Hirsen, Dinkel, Tapioka;
c) einen oder mehrere nicht transferierbare Wirkstoffe zum Regenerieren strukturell / funktionell veränderter Lipide, ausgewählt aus wasserlöslichen oder wasserdispergierbaren Wirkstoffen der Gruppe aus Erythorbinsäure, Gluthation, alpha-Liponsäure, pflanzlichen Phenolverbindungen, höhermolekularen Enzymproteinen, Kofaktoren, wasserlöslichen Antioxidantien, Redoxsystemen;
d) ggf. einen oder mehrere anwendungsbezogene Zusatzstoffe.

2. Zusammensetzungen nach Anspruch 1, **dadurch gekennzeichnet, dass** die strukturell / funktionell veränderten Lipide oxidativ veränderte Lipide sind und die Lipide im Lipiddepot oxidativ nicht veränderte Lipid(e) sind.

3. Zusammensetzungen nach einem der Ansprüche 1 oder 2, **dadurch gekennzeichnet, dass** das oder die Recycling-Transferproteine Transferraten für oxidativ veränderte Lipide aufweisen.

4. Zusammensetzungen nach einem der Ansprüche 1 bis 3, **dadurch gekennzeichnet, dass** sie weiterhin ein oder mehrer Transferproteine mit Transferraten für nicht strukturell / funktionell, insbesondere nicht oxidativ veränderte Lipide aufweisen.

5. Zusammensetzungen nach Anspruch 1 bis 4, **dadurch gekennzeichnet, dass** das Lipiddepot in Form liposamaler Lipidstrukturen, umfassend unilamellare oder multilamellare Liposomen, enthalten ist.

6. Zusammensetzungen nach Anspruch 1 bis 5, **dadurch gekennzeichnet, dass** der nicht transferierbare Wirkstoff c) ausgewählt ist aus NADH, Peroxidasen, Erythorbinsäure, Gluthation, alpha-Liponsäure, Gluthathion, Grüntee- Extrakt, Cystus-Tee-Extrakt, Apfelquercetin, Heidelbeerextrakt, Holunderbeer-Extrakt oder Mischungen hiervon.

7. Zusammensetzungen nach Anspruch 6, **dadurch gekennzeichnet, dass** das der nicht transferierbare Wirkstoff c) ausgewählt ist aus, Erythorbinsäure, Gluthation, Cystus-Tee-Extrakt, Apfelquercetin oder Mischungen hiervon.

8. Zusammensetzungen nach einem der Ansprüche 1 bis 7, **dadurch gekennzeichnet, dass** 0,01 bis 10 Gew. % eines oder mehrerer Recycling Transferproteine b) 1 bis 40 Gew. % des Lipiddepots a), 0,01 bis 10 Gew. % eines oder mehrerer Wirkstoffe c) und d) Zusatzstoffe, ausgewählt aus 0,1 bis 20 Gew.% Additiven und (als Rest) Wasser enthalten sind.

9. Zusammensetzungen nach einem der Ansprüche 1 bis 8, **dadurch gekennzeichnet, dass** Zusatzstoffe für die topische Anwendung enthalten sind.

10. Zusammensetzungen nach einem der Ansprüche 1 bis 9, **dadurch gekennzeichnet, dass** als Zusatzstoffe ein oder mehrere Stoffe, ausgewählt aus Wasser und Additiven gewählt aus Kolloiden, Konsistenzreglem, grenzflächenaktiven Stoffen, Konservierungsmittel, Zusatzwirkstoffen, Parfümstoffen, Farbstoffen, Pflegestoffen oder Mischungen hiervon enthalten sind.

11. Zusammensetzungen nach Anspruch 10, **dadurch gekennzeichnet, dass** die grenzflächenaktiven Stoffe ausgewählt sind aus O/W-, W/O-, Co- Emulgatoren, Co- Tensiden oder Mischungen hiervon.

12. Zusammensetzungen gemäß Anspruch 10 oder 11, **dadurch gekennzeichnet, dass** sie in Form einer flüssigen, halbflüssigen, festen Form als O/W Emulsion, W/O Emulsion, einer Gelemulsion, Gel, Creme, Balsam, Lotion, Milch, Gesichtsmaske, Spray, Badezusatz oder Duschzusatz vorliegen.

13. Verfahren zur Regenerierung von oxidativ veränderten Lipidaggregaten, **dadurch gekennzeichnet, dass** man eine Zusammensetzung gemäß einem der Ansprüche 1 bis 12 two kosmetisch oder kosmetish-balneologischen Applikation auf die Oberfläche mit den zu behandelten Lipidaggregaten in Kontakt bringt

14. Verfahren gemäß Anspruch 13, **dadurch gekennzeichnet, dass** durch Applikation einer Zusammensetzung gemäß einem der Ansprüche 1 bis 12, oxidativ veränderte Lipide aus den diese enthaltenden Lipidaggregaten exportiert, im Depot a) mit Hilfe eines oder mehrerer Wirkstoffe c) recyclet und danach in die Lipidaggregate zurücktransferiert werden.

15. Verfahren gemäß Anspruch 13 oder 14, **dadurch gekennzeichnet, dass** es sich bei den Lipidaggregmen um Haut von Menschen und um eine kosmetische Regenerierung handelt.

16. Verwendung von Zusammensetzungen gemäß einem der Ansprüche 1 bis 12 zur topischen kosmetischen oder kosmetisch- balneologischen regenerativen Pflege oder regenerativen Pflegereinigung der Haut von Säugern, insbesondere von Menschen.

17. Verwendung von Zusammensetzungen gemäß einem der Ansprüche 1 bis 12 zur Herstellung eines Mittels zur prophylaktischen, dermatologischen, therapeutischen regenerativen Behandlung der Haut von Säugern.

18. Verwendung von Zusammensetzungen gemäß einem der Ansprüche 1 bis 12 zur Herstellung eines Mittels zum gezielten Engineering von Biomembranen in vivo.

19. Verwendung von Zusammensetzungen gemäß einem der Ansprüche 1 bis 12 zum gezielten Engineering von Biomembranen ex vivo, in vitro.

20. Verwendung von Zusammensetzungen gemäß einem der Ansprüche 1 bis 12 zum gezielten Engineering von künstlich hergestellten Lipid- Membranen

## Claims

1. Compositions comprising:
a) a depot of one or more structurally/functionally unmodified lipids, selected from natural or synthetic fats, oils, waxes, fatty alcohols, fatty acid esters, fatty acid partial esters, glycerides, silicone oils, silicone waxes, hydrocarbons, lecithins, sphingolipids, cholesterols, phospholipids, gangliosides, cerebrosides, ceramides or mixtures thereof;
b) one or more recycling transfer proteins with transfer rates for structurally/functionally modified, especially oxidised lipids which exhibit a transfer rate of 600 to 8000 ng/min/mg proteins and an average molecular mass of 3500 to 8000 D and which are selected from proteins from buckwheat, millet, spelt, tapioca;
c) one or more non-transferable active substances for regenerating structurally/functionafly modified lipids selected from water-soluble or water-dispersible active substances from the group of erythorbic acid, gluthatione, alpha-liponic acid, vegetal phenol compounds, higher molecular weight enzyme proteins, cofactors, water-soluble antioxidants, redox systems,
d) optionally one or more application-oriented additives.

2. Compositions according to claim 1, **characterised in that** the structurally/functionally modified lipids are oxidatively modified lipids and the lipids in the lipid depot are oxidatively unmodified lipid(s).

3. Compositions according to one of claims 1 or 2, **characterised in that** the recycling transfer protein(s) exhibit transfer rates for oxidatively modified lipids.

4. Compositions according to one of claims 1 to 3, **characterised in that** they further exhibit one or more transfer proteins with transfer rates for non-structurally/functionally, especially non-oxidatively, modified lipids.

5. Compositions according to claim 1 to 4, **characterised in that** the lipid depot is comprised in the form of liposomal lipid structures, containing unilamellar or multilamellar liposomes.

6. Compositions according to claim 1 to 5, **characterised in that** the non-transferable active substance c) is selected from NADH, peroxidases, erythorbic acid, gluthatione, alpha-liponic acid, gluthathione, green tee extract, cystus tea extract, apple quercetin, blueberry extract, elderberry extract or mixtures thereof.

7. Compositions according to claim 6, **characterised in that** the non-transferable active substances c) is selected from erythorbic acid, gluthatione, cystus tea extract, apple quercetin or mixtures thereof.

8. Compositions according to one of claims 1 to 7, **characterized in that** they comprise 0.01 to 10 wt % of one or more recycling transfer proteins b) 1 to 40 wt % of the lipid depot a), 0.01 to 10 wt % of one or more active substances c) and d) adjuvants, selected from 0.1 to 20 wt % additives and (as the remainder) water.

9. Compositions according to one of claims 1 to 8, **characterized in that** they comprise adjuvants for the topical application

10. Compositions according to one of claims 1 to 9, **characterized in that** they comprise as the adjuvants one or more substances, selected from water and additives selected from colloids, consistency regulators, surface-active substances, preservatives, additional active substances, fragrances, dyes, skin care agents or mixtures thereof.

11. Compositions according to claim 10, **characterised in that** the surface-active substances are selected from O/W- W/O-, co-emulsifiers, co-surfactants or mixtures thereof.

12. Compositions according to claim 10 or 11, **characterised in that** they are present in the form of a liquid, semi-liquid, solid form as an O/W emulsion, W/O emulsion, a gel emulsion, gel, cream, balm, lotion, milk, face mask, spray, bath additive or shower additive.

13. Method for regenerating oxidatively modified lipid aggregates, **characterised in that** a composition according to one of claims 1 to 12 is brought into contact on the surface with the lipid aggregates to be treated for the cosmetic or cosmetic-balneological application.

14. Method according to claim 13, **characterised in that** by application of a composition according to one of claims 1 to 12, oxidatively modified lipids are exported from the lipid aggregates containing them, recycled in depot a) with the help of one or more active ingredients c) and then transferred back into the lipid aggregates.

15. Method according to claim 13 or 14, **characterised in that** as regards the lipid aggregates it relates to human skin and a cosmetic regeneration.

16. Use of compositions according to one of claims 1 to 12 for topical cosmetic or cosmetic-balneological regenerative skin care or regenerative gentle cleansing of the skin of mammals, especially of humans.

17. Use of compositions according to one of claims 1 to 12 for the production of an agent for the prophylactic, dermatological, therapeutic regenerative treatment of the skin of mammals.

18. Use of compositions according to one of claims 1 to 12 for the production of an agent for the selective engineering of biomembranes in vivo.

19. Use of compositions according to one of claims 1 to 12 for the selective engineering of biomembranes ex vivo, in vitro.

20. Use of compositions according to one of claims 1 to 12 for the selective engineering of synthetically produced lipid membranes.

## Revendications

1. Compositions, comprenant :
a) un dépôt d'un ou de plusieurs lipides structurellement/fonctionnellement non modifiés, choisis parmi les graisses, les huiles, les cires, les alcools gras, les esters d'acide gras, les esters partiels d'acide gras, les glycérides, les huiles de silicone, les cires de silicone, les hydrocarbures, les lécithines, les sphingolipides, les cholestérols, les phospholipides, les gangliosides, les cérébrosides, les céramidies, naturels ou synthétiques, ou leurs mélanges
b) une ou plusieurs protéines de transfert et de recyclage présentant des vitesses de transfert pour des lipides structurellement/fonctionnellement modifiés, en particulier oxydés, qui présentent une vitesse de transfert de 600 à 8000 ng/min/mg de protéine et une masse moléculaire moyenne de 3500 à 8000 D et qui sont choisies parmi les protéines de sarrasin, de millet, d'épeautre, de tapioca ;
c) une ou plusieurs substances actives non transférables pour la régénération de lipides structurellement/fonctionnellement modifiés, choisies parmi les substances actives solubles ou dispersibles dans l'eau du groupe formé par l'acide érythorbique, le gluthation, l'acide alpha-liponique, les composés phénoliques végétaux, les protéines enzymatiques de poids moléculaire plus élevé, les cofacteurs, les antioxydants solubles dans l'eau, les systèmes redox ;
d) le cas échéant un ou plusieurs additifs relatifs à l'utilisation.

2. Compositions selon la revendication 1, **caractérisées en ce que** les lipides structurellement/fonctionnellement modifiés sont des lipides modifiés par oxydation et les lipides dans le dépôt de lipides sont des lipides non modifiés par oxydation.

3. Compositions selon l'une quelconque des revendications 1 ou 2, **caractérisées en ce que** la ou les protéines de transfert et de recyclage présentent des vitesses de transfert pour des lipides modifiés par oxydation

4. Compositions selon l'une quelconque des revendications 1 à 3, **caractérisées en ce qu'**elles présentent en outre une ou plusieurs protéines de transfert présentant des vitesses de transfert pour des lipides structurellement/fonctionnellement non modifiés, de préférence non modifiés par oxydation.

5. Compositions selon la revendication 1 à 4, **caractérisées en ce que** le dépôt de lipides est contenu sous forme de structures lipidiques liposomales, comprenant des liposomes unilamellaires ou multilamellaires.

6. Compositions selon la revendication 1 à 5, **caractérisées en ce que** la substance active c) non transférable est choisie parmi le NADH, les peroxydases, l'acide érythorbique, le glutathion, l'acide alpha-liponique, gluthathion, l'extrait de thé vert, l'extrait de thé de cistus, la quercétine de pomme, l'extrait de myrtille, l'extrait de baie de sureau ou leurs mélanges.

7. Compositions selon la revendication 6, **caractérisées en ce que** la substance active c) non transférable est choisie parmi l'acide érythorbique, le gluthation, l'extrait de thé de cistus, la quercétine de pomme ou leurs mélanges.

8. Compositions selon l'une quelconque des revendications 1 à 7, **caractérisées en ce qu'**elles contiennent 0,01 à 10% en poids d'une ou de plusieurs protéines de transfert et de recyclage b), 1 à 40% en poids du dépôt de lipides a), 0,01 à 10% en poids d'une ou de plusieurs substances actives c) et d) des adjuvants choisis parmi 0,1 à 20% en poids d'additifs et de l'eau (comme reste).

9. Compositions selon l'une quelconque des revendications 1 à 8, **caractérisées en ce qu'**elles contiennent des adjuvants pour l'utilisation topique.

10. Compositions selon l'une quelconque des revendications 1 à 9, **caractérisées en ce qu'**elles contiennent comme adjuvant une ou plusieurs substances choisies parmi l'eau et les additifs choisis parmi les colloïdes, les agents de régulation de la consistance, les substances tensioactives, les agents de conservation, les substances actives supplémentaires, les parfums, les colorants, les agents de soin ou leurs mélanges.

11. Compositions selon la revendication 10, **caractérisées en ce que** les substances tensioactives sont choisies parmi les émulsifiants HIE, E/H, les co-émulsifiants, les co-agents tensioactifs ou leurs mélanges.

12. Compositions selon la revendication 10 ou 11, **caractérisées en ce qu'**elles se trouvent sous une forme liquide, semi-liquide, solide en tant qu'émulsion HIE, émulsion E/H, émulsion de gel, gel, crème, baume, lotion, lait, masque pour le visage, spray, adjuvant de bain ou de douche.

13. Procédé pour la régénération d'agrégats lipidiques modifiés par oxydation, **caractérisé en ce qu'**on met en contant une composition selon l'une quelconque des revendications 1 à 12 pour l'application cosmétique ou cosmétique-balnéologique sur la surface avec les agrégats lipidiques à traiter.

14. Procédé selon la revendication 13, **caractérisé en ce que** par l'application d'une composition selon l'une quelconque des revendications 1 à 12, des lipides modifiés par oxydation sont exportés des agrégats lipidiques les contenant, recyclés dans le dépôt a) à l'aide d'une ou de plusieurs substances actives c) puis transférés en retour dans les agrégats lipidiques.

15. Procédé selon la revendication 13 ou 14, **caractérisé en ce qu'**il s'agit, pour les agrégats lipidiques, de la peau d'êtres humains et d'une régénération cosmétique.

16. Utilisation de compositions selon l'une quelconque des revendications 1 à 12 pour le soin de régénération ou le nettoyage et soin régénérateur topique cosmétique ou cosmétique-balnéologique de la peau de mammifères, en particulier d'êtres humains.

17. Utilisation de compositions selon l'une quelconque des revendications 1 à 12 pour la préparation d'un agent destiné au traitement de régénération prophylactique, dermatologique, thérapeutique de la peau de mammifères.

18. Utilisation des compositions selon l'une quelconque des revendications 1 à 12 pour la préparation d'un agent destiné à l'ingénierie ciblée de biomembranes in vivo.

19. Utilisation des compositions selon l'une quelconque des revendications 1 à 12 pour l'ingénierie ciblée de biomembranes ex vivo, in vitro,

20. Utilisation de compositions selon l'une quelconque des revendications 1 à 12 pour l'ingénierie ciblée de membranes lipidiques préparées artificiellement.
